# EUROPÄISCHE PATENTSCHRIFT

(11) **EP 0 955 361 B1**
(45) Veröffentlichungstag und Bekanntmachung des Hinweises auf die Patenterteilung: **18.01.2006**
(21) Anmeldenummer: 99103484.4
(22) Anmeldetag: 22.02.1991
(51) Int. Cl.: C12N 5/20, C07K 16/30, A61K 39/395, G01N 33/577, G01N 33/574

(54) **Monoklonale Antikörper gegen tumorassoziierte Antigene, Verfahren zu ihrer Herstellung sowie ihre Verwendung**
Monoclonal antibodies to tumor-associated antigens, methods for their production, and their use
Anticorps monoclonaux contre des antigènes associés aux tumeurs, procédés de préparation et utilisation

(30) Priorität: 22.02.1990 DE 4005630
(43) Veröffentlichungstag der Anmeldung: 10.11.1999
(62) Teilanmeldung aus: 91102608.6
(73) Patentinhaber: Dade Behring Marburg GmbH, 35001 Marburg (DE)
(72) Erfinder: Bosslet, Klaus, Dr., 13465 Berlin (DE); Auerbach, Bernhard, Dr., 35041 Marburg (DE); Peters, Helmut, Dr., 35043 Marburg (DE)

(56) Entgegenhaltungen:
- R. KIBBELAAR ET AL.: "Expression of the embryonal neural cell adhesion molecule N-CAM in lung carcinoma. Diagnostic usefulness of monoclonal antibody 735 for the distinction between small cell lung cancer and non-small cell lung cancer." JOURNAL OF PATHOLOGY, Bd. 159, Nr. 1, September 1989 (1989-09), Seiten 23-28, XP002112647 Edinburgh, Grossbritannien
- J. WATANABE ET AL.: "Monoclonal antibody that distinguishes small-cell lung cancer from non-small cell lung cancer." CANCER RESEARCH, Bd. 47, Nr. 3, 1. Februar 1987 (1987-02-01), Seiten 826-829, XP002112648 Baltimore, MD, VSA
- C. MOOLENAAR ET AL.: "Expression of neural cell adhesion molecule-related sialoglycoprotein in small cell lung cancer and neuroblastoma cell lines H69 and CHP-212." CANCER RESEARCH, Bd. 50, Nr. 4, 15. Februar 1990 (1990-02-15), Seiten 1102-1106, XP002112649 Baltimore, MD, VSA
- B. FUCHS ET AL.: "Biochemical and immunochemical analysis of gangliosides of human small cell lung carcinoma: Production of monoclonal antibodies against a unique marker of small cell lung carcinoma, ganglioside Fuc GM." BIOTECHNOLOGY AND APPLIED BIOCHEMISTRY, Bd. 10, Nr. 3, Juni 1988 (1988-06), Seiten 273-286, XP002112650 Orlando, FL, VSA

## Beschreibung

Die Erfindung betrifft monoklonale Antikörper (MAK) und deren Fragmente, die an definierte tumorassoziierte Antigene vornehmlich vom kleinzelligen Lungenkarzinom (SCLC = small cell lung carcinoma), vom Melanom, vom Neuroblastom und weiteren Tumoren neuroektodermalen Ursprungs binden, Hybridomazellen zu ihrer Herstellung sowie die mit Hilfe dieser Antikörper bzw. Antikörperfragmente definerbaren und/oder isolierbaren Antigene. Die Antikörper, Antikörperfragmente und Antigene können als Diagnostikum, Wirkstoff oder Wirkstoffträger verwendet werden.

Die Identifizierung, Charakterisierung und Therapie von Tumoren gehört zu den wichtigsten Gebieten der Diagnostik und Therapie. Durch die Möglichkeit, monoklonale Antikörper hoher Spezifität zu produzieren, hat die Entwicklung auf diesem Gebiet große Fortschritte gemacht. Als besonders wichtig hat sich dabei die Identifizierung von sogenannten Tumormarkern erwiesen. Unter Tumormarker versteht man Produkte der Tumorzelle, z. B. tumorassoziierte Antigene, aber auch Substanzen, die vom gesunden Gewebe als Reaktion des Organismus auf das maligne Wachstum gebildet werden. Bekannte Tumormarker sind z. B. das CEA, das AFP, aber auch durch monoklonale Antikörper definierte Tumorantigene, wie z. B. das CA 19-9 oder das CA 125.

Das Haupteinsatzgebiet der Tumormarker in der In-vitro-Diagnostik besteht in der Therapie sowie der Verlaufskontrolle bei Tumorpatienten. Bestimmte Tumormarker können auch für die Differentialdiagnose oder für das Screening von Risikogruppen eingesetzt werden.

Zur Identifizierung von kleinzelligen Bronchialkarzinom (SCLC) sind schon eine Reihe von Versuchen unternommen worden. So ist zum Beispiel bekannt, daß die Neuron-spezifische Enolase, ein Isoenzym der Enolase (EC 4.2.1.11), von malignen Tumoren neuroektodermalen Ursprungs, wie z. B. dem kleinzelligen Bronchialkarzinom oder dem Neuroblastom vermehrt gebildet wird und bei Tumorpatienten in erhöhten Konzentrationen im Serum auftritt.

Es hat sich aber gezeigt, daß ein Teil der an den o.g. Tumoren erkrankten Patienten falsch-negativ erfaßt wird. Da rote Blutkörperchen, aber auch Thrombozyten, größere Mengen an NSE enthalten, ist es ferner der Fall, daß durch deren Lyse falsch erhöhte NSE-Serum- oder Plasmaspiegel und somit falsch-positive Werte gemessen werden (Pahlman et al., Tumour Biology 5: 127 - 139, 1984).

Aus der Patentanmeldung EP 0 323 802 ist ein monoklonaler Antikörper gegen ein Zelloberflächenantigen von Lungenkarzinomen bekannt. Aus MAIER et al. (Br.J. Cancer, 1989, 59, 692 - 695) ist aber bekannt, daß der in der EP 0 323 802 verwendete Antikörper SWA 20 ein Epitop (Cluster 5) erkennt, das nur bei 45 % der untersuchten SCLC-Proben eine mittel bis stark positive Reaktion zeigte.

Es ist daher wünschenswert, einen anderen von der NSE unabhängigen, spezifischen Tumormarker für das Neuroblastom bzw. das kleinzellige Lungenkarzinom zu produzieren.

Erfindungsgemäß werden nun monoklonale Antikörper gegen ein tumorassoziiertes Antigen vorgeschlagen, wobei das Antigen von Tumoren vornehmlich aus der Gruppe der neuroektodermalen Tumoren, wie z. B. dem kleinzelligen Lungenkarzinom (SCLC), dem Melanom, dem Neuroblastom sowie aus dem Kulturüberstand von Zellinien aus diesen Tumoren stammt, insbesondere Antigene aus SCLC-Zellinien, die in der nichtreduzierenden SDS-PAGE ein Molekulargewicht von 170 ± 10 kDa, 140 ± 10 kDa, 105 ± 10 kDA, 67 ± 10 kDa und 50 ± 10 kDA aufweisen oder daß das Antigen aus Körperflüssigkeiten von Tumorpatienten stammt, insbesondere Antigene aus dem Serum von SCLC-Patienten, die in der nichtreduzierenden SDS-PAGE Molekulargewichte von 220-260 kDa und 160-200 kDa aufweisen. Diese Banden werden mit dem MAK BW SCLC-1 im Western blot nachgewiesen und bei 5 von 5 SCLC-Tumorpatienten gefunden. Bei 2 von 4 Normalseren wurde keine Bande, bei 2 ganz schwache Banden in der gleichen Position wie in den Tumorseren gefunden.

Die erfindungsgemäßen monoklonalen Antikörper, binden an dasselbe Editor wie der Referenzantikörper MAK BW SCLC-1.

Besonders bevorzugt sind dabei solche monoklonalen AntiKörper, die von der Hybridomazellinien BW SCLC-1 produziert werden.

Die Erfindung betrifft ferner die Hybridomazellinie, die den erfindungsgemäßen monoklonalen Antikörper produziert, d.h die Hybridomazellinien BW SCLC-1 (ECACC Deposit Nr 90 022 110).

Unter monoklonalen Antikörpern werden im Sinne dieser Erfindung auch Antikörperfragmente, wie z. B. Fab und F(ab)₂ und Derivate verstanden.
Die Hybridomazellinien BW SCLC-1 und 2, die die monoklonalen Antikörper MAK BW SCLC-1 und MAK BW SCLC-2 produzieren, sind unter der Nummer 90 022 110 bzw. 90 022 109 am 21. Februar 1990 bei der European Collection of Animal Cell Cultures (ECACC) hinterlegt worden.

Monoklonale Antikörper können nach dem Fachmann an sich bekannten Verfahren hergestellt werden, wobei bevorzugterweise Antigene aus dem Überstand von SCLC-Zellinien, die in der nichtreduzierenden SDS-PAGE ein Molekulargewicht von 170 ± 10 kDa, 140 ± 10 kDa, 105 ± 10, 67 ± 10 kDa oder 50 ± 10 kDa aufweisen, zum Immunisieren verwendet werden. Die Erfindung betrifft auch Antigene, die durch Immunadsorption an einen Antikörper gemäß Anspruch 1 gebunden werden können.

Unter Immunadsorption im Sinne der Erfindung verstehen wir dem Fachmann an sich bekannte Isolierungsverfahren, bei denen mindestens ein Reinigungsschritt auf einer immunchemischen Reaktion mit einem der erfindungsgemäßen Antikörper gemäß Anspruch 1, bevorzugterweise gemäß Anspruch 2, beruht. Die Abtrennung des Ak-Ag Komplexes kann dabei auf dem Fachmann an sich bekannte Weise, z. B. durch Bindung des Antikörpers an eine Festphase erfolgen.

Die Erfindung betrifft auch die Verwendung eines erfindungsgemäßen Antigens zur Erzeugung einer Immunantwort in Säugetieren, wobei der Mensch dabei ausdrücklich eingeschlossen ist.

Die Erfindung betrifft auch die Verwendung der erfindungsgemäßen Antikörper und/oder Antigene in Diagnostik und/oder Therapie.

In der Diagnostik werden bevorzugt Antikörper in dem Fachmann an sich bekannten immunchemischen heterogenen oder homogenen Bestimmungsmethoden eingesetzt, wobei bei den homogenen Verfahren die Partikel-verstärkte Nephelometrie oder Turbidimetrie bevorzugt ist. Bei den heterogenen Immunoassays wird der festphasengebundene Sandwichassay bevorzugt, wobei die Festphase bevorzugterweise ein Polystyrolröhrchen, ein Latexpartikel, ein magnetisierbarer Partikel oder eine flächenförmige Festphase ist.
Bevorzugt ist ein diagnostisches Verfahren zum Nachweis eines tumorassoziierten Antigens, wobei ein erfindungsgemäßer Antikörper als spezifischer Bindungspartner eingesetzt wird.

Die erfindungsgemäßen Antikörper und Antigene können auch in Biosensoren eingesetzt werden. Biosensoren sind dem Fachmann an sich bekannt. Besonders bevorzugt ist ein Verfahren, wobei ein zweiter spezifischer Bindungspartner, wie z. B. ein Antikörper, ein Lectin oder ein Rezeptor eingesetzt wird.

Ganz bevorzugt ist dabei ein Verfahren, wobei der zweite spezifische Bindungspartner Sialinsäure, Polysialinsäure oder α(2-8)-verknüpfte N-Acetyl-Neuraminsäure spezifisch erkennt.

Zum Nachweis und zur Quantifizierung kann dabei einer der spezifischen Bindungspartner ein nachweisbares Label tragen. Diese Label sind dem Fachmann an sich bekannt und können z. B. ein Chromophor, ein Luminophor, ein Fluorophor, ein Enzym, ein radioaktives Isotop oder ein gefärbter oder auch ungefärbter Partikel sein.
Bevorzugt ist ein Verfahren, wobei der nicht-markierte spezifische Bindungspartner, nach dem Fachmann an sich bekannten Verfahren, direkt oder indirekt, z. B. über einen weiteren Antikörper odr eine Biotin-Avidin Brücke an eine Festphase gekoppelt ist.

Besonders bevorzugt sind ferner die in den Beispielen beschriebenen Ausführungsformen.

Die MAK BW SCLC-1 und -2 können aufgrund ihrer immunhistochemischen Bindung an menschliche Normalgewebe und Tumore als SCLC cluster 1 MAK bezeichnet werden (Souhami et al., LANCET 8. August 1987, S. 325 - 326). Dieses Cluster enthält MAK, die optimal an kleinzellige Lungenkarzinome binden. Desweiteren binden diese MAK an neurales Gewebe, Neuroblastome und einige Melanome.

Patel et al. (Int. J. Cancer 44: 573 - 578, 1989) haben gezeigt, daß diese Cluster 1 MAK N-CAM erkennen und zwar hauptsächlich die 140 und 180 kDa Isoformen (Patel et al., Int. J. Cancer 44: 1062 - 1068, 1989). Es ist bisher noch nicht beschrieben worden, daß das N-CAM und insbesondere die 160 - 180, 130 - 150, 95 - 115 , 57 - 77 bzw. 40 - 60 kDa (Kulturüberstand) sowie die 220 - 260 kDa und 160 bis 200 kDa (Serum) iso-Form von Tumorzellen aktiv ausgeschieden werden und somit als Tumormarker genutzt werden können. Da das N-CAM u. a. auch im Nerven-, Muskel- und Nierengewebe nachweisbar ist (Roth et al., Proc.Natl.Acad.Sci, USA 85, 2999 - 3003, 1988; Roth et al., Virchows Archiv B 56, 95 - 102, 1988), kann erwartet werden, daß es auch bei anderen krankhaften Prozessen, insbesondere diese Gewebe betreffend, zu Veränderungen der N-CAM-Konzentration in den Körperflüssigkeiten der Patienten kommen kann, so daß N-CAM auch für diese Krankheiten als diagnostischer Marker dienen kann.

Die Spezifität der MAK BW SCLC-1 und -2 läßt sich nicht nur für eine immunhistochemische Differenzierung von verschiedenen Tumorgeweben und Normalgeweben nutzen, sondern überraschenderweise erwies sich eine Kombination eines Anti-N-CAM-MAKs als Festphasenantikörper der α-(2-8)-verknüpfte N-Acetyl-Neuraminsäure (Finne et al., J. Immunol. 138: 4402 - 4407, 1987; Häyrinen et al., Molecular Immunology 26: 523 - 529, 1989) erkennt, mit den MAK BW SCLC-1 und -2 als Konjugatantikörpern als besonders gut geeignet, um einen Tumormarker-Immunoassay zu entwickeln. Mit diesem Assay wurde nachgewiesen, daß die erkannten Antigene im Serum bzw. Plasma von Patienten mit SCLC und Neuroblastom häufig in einer deutlich gegenüber Serum oder Plasma gesunder Kontrollpersonen erhöhten Konzentration vorliegen. Hieraus läßt sich eine gute sensitivität des Testes für die erwähnten Tumore ableiten.

Die Antikörper BW SCLC-1 und -2 oder deren Fragmente können auch nach dem Fachmann bekannten Verfahren radioaktiv markiert werden, um sie für die Immunszintigraphie bzw. auch für die Immuntherapie einzusetzen. Zusätzlich könnten diese monoklonalen Antikörper als Wirkstoffträger, z. B. für Zelltoxine, eingesetzt und zur Therapie maligner Erkrankung genutzt werden. Auch die Erzeugung von Antikörperkonstrukten, z. B. durch Einsetzen der hypervariablen Regionen in ein humanes MAK-Gerüst, ist nach der Analyse der vollständigen Nukleotidsequenz der V-Gene der MAK BW SCLC-1 und -2 technisch möglich (Jones et al., Nature 321: 522 - 525, 1986; Verhoeyen et al., Science 239: 1534 - 1536, 1988).

Die erfindungsgemäßen Antigene können auch zur Herstellung eines aktiven Impfstoffes, bzw. geeignete Antikörper zur Herstellung eines passiven Impfstoffes verwendet werden.

Die folgenden Beispiele dienen der Erläuterung der Erfindung und schränken sie in keiner Weise ein.

### Beispiel 1

### Erzeugung der monoklonalen Antikörper BW SCLC-1 und -2

Als Immunogen wurden die humanen kleinzelligen Lungenkarzinom-zell-Linien GOT und MR 22 benutzt. Ihre Kultivierung in vitro erfolgte als Suspensionskultur in Basalmedium (DMEM), welches mit 10 % Rinderserum versetzt ist. Balb/c-Mäuse wurden mit 3 x in Salzlösung (PBS) gewaschenen Zellen entsprechend dem nachfolgenden Schema immunisiert:

| Injektionstag | Zellzahl/Maus | Route | Adjuvans | Zelltyp |
|---|---|---|---|---|
| 0 | 1,5 x 10⁷ | S.C. | CFA | MR 22 |
| 7 | 1 x 10⁷ | S.C. | CFA | GOT |
| 14 | 1 x 10⁷ | S.C. | IFA | MR 22 |
| 21 | 1 x 10⁷ | S.C. | IFA | GOT |
| 28 | 1 x 10⁷ | S.C. | IFA | MR 22 |
| 32 | 2 x 10⁶ | i.v. | PBS | GOT |
| 33 | 2 x 10⁶ | i.v. | PBS | MR 22 |

| | | | | |
|---|---|---|---|---|
| (Abkürzungen: CFA = komplettes Freund's Adjuvans, | | | | |
| IFA = inkomplettes Freund's Adjuvans | | | | |
| S.C. = subkutan | | | | |
| i.v. = intravenös) | | | | |

Die Milzen der so immunisierten Mäuse wurden am Tag 35 entnommen und in einem Verhältnis von 6 : 1 (Milzzellen zu Myelomzellen) mit der SP-2 Myelomzell-Linie (Shulman et al., Nature 276: 269, 1978) nach der von Köhler und Milstein beschriebenen Technik fusioniert (Köhler u. Milstein, Nature 256: 495, 1975).

Die in dem Zeitraum von 8 - 28 Tagen ausgewachsenen Hybride wurden mittels zytofluorometrischer Analyse getestet, ob sie MAK ausscheiden, die an die GOT und die MR 22 SCLC-Zelllinien binden. Positive Hybride wurden mittels der limited dilution Technik 3 x kloniert und die von diesen Subklonen produzierten MAK verschie-denen immunologischen Testverfahren unterworfen. Hybride, die aufgrund dieser immunologischen Tests besonders interessante MAK ausschieden, wurden in flüssigem Stickstoff eingefroren und unter der Bezeichnung BW SCLC-1 bzw. BW SCLC-2 bei der ECACC unter der Deposit-No. 90 022 110 bzw. 90 022 109 hinterlegt. Die von diesen Hybriden ausgeschiedenen MAK werden als MAK BW SCLC-1 oder MAK BW SCLC-2 bezeichnet.

### Beispiel 2

### Immunhistochemische Charakterisierung der Spezifität der MAK BW SCLC-1 und MAK BW SCLC-2

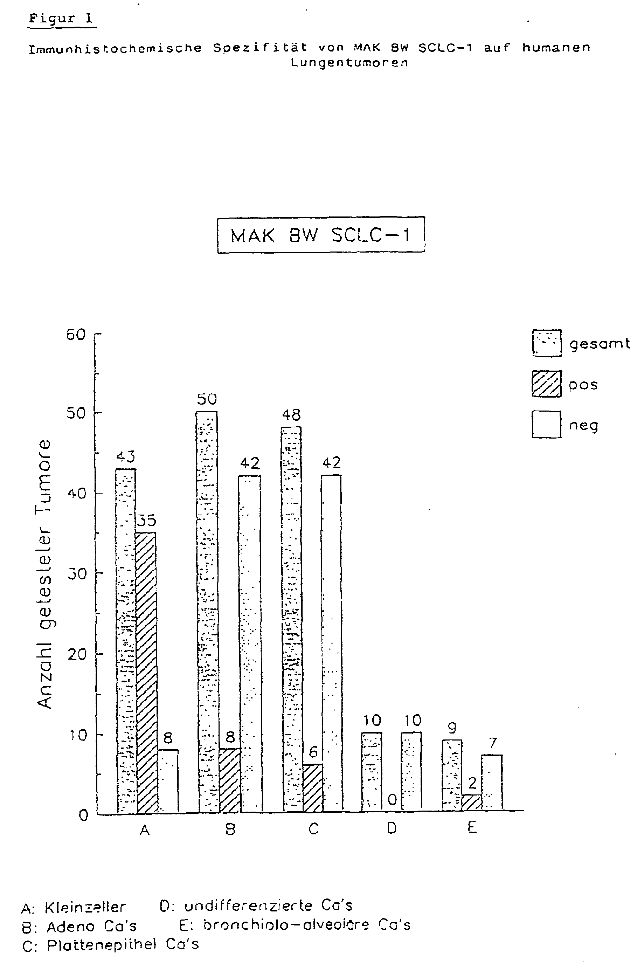

Mittels der APAAP-Technik (Cordell et al., J. Histochem. Cytochem. 32: 219, 1984) wurde die Expression der Epitope, die von beiden MAK erkannt wurden, auf kryopräservierten menschlichen Normalgeweben und Tumoren bestimmt. Hierbei stellte sich heraus, daß die Expression auf Tumore neuroektodermalen Ursprungs beschränkt ist, d. h. mehr als 80 % der kleinzelligen Lungentumore (Fig. 1), Neuroblastome und Hirntumore waren deutlich positiv (Tab. 1), zusätzlich ein Großteil der Melanome. Die meisten anderen nicht vom Neuroektoderm abgeleiteten Tumore waren negativ (s. Tab. 1). Die Reaktionen des MAK BW SCLC-1 mit menschlichen kryopräservierten Normalgeweben sind in Tab. 2 zusammengefaßt. Der MAK BW SCLC-2 zeigt ein vergleichbares Reaktionsmuster. Lediglich seine Bindung an Knochenmark ist stärker ausgeprägt.

### Beispiel 3

### Charakterisierung der von den MAK BW SCLC-1 und MAK BW SCLC-2 erkannten Antigene und Epitope

Der MAK BW SCLC-1 wurde über Protein-A affinitätschromatographisch gereinigt und ebenso wie der gegen α-(2-8)-verknüpfte N-Acetyl-Neuraminsäure gerichtete MAK 735 an CN-Br aktivierte Sepharose gekoppelt (Ey et al., Immunochemistry 15: 429, 1978; Pharmacia Fine Chemicals, Affinity Chromatography, Principles and Methods, Seite 15 - 18, 1979).
Zellkulturmedien, in denen die GOT Zell-Linie kultiviert wurde, wurden über die mit MAK BW-SCLC-1 beladene CN-Br aktivierte Sepharosesäule gepumpt und das spezifisch bei pH 7 gebundene Antigenmaterial bei pH 2,5 eluiert. Das so erhaltene Eluat wurde mittels SDS-Polyacrylamidgelelektrophorese (SDS-PAGE) sowohl unter reduzierenden als auch nicht reduzierenden Bedingungen aufgetrennt, anschließend nach dem Fachmann bekannten Verfahren einer Silberfärbung unterzogen bzw. auf Nitrozellulose transferiert (Western blot) und immunchemisch auf das Vorhandensein von Antigenen der MAK BW SCLC-1 oder -2 sowie anderer MAK bekannter Spezifität untersucht.

Hierbei wurden folgende Beobachtungen gemacht:
a) Sowohl die von MAK BW SCLC-1 als auch die von MAK BW SCLC-2 erkannten Antigene kommen in überständen Epitop-positiver kleinzelliger Lungenkarzinomzell-Linien vor.
b) Das Molekulargewicht der Antigene liegen in der nicht reduzierenden SDS-PAGE bei 170 ± 10 kDa, 140 ± 10 kDa, 105 ± 10 kDa, 67 ± 10 kDa und 50 ± 10 kDa. Die Breite der Banden deuten auf Glykoproteine hin. Unter reduzierenden Bedingungen werden die Antigene von MAK BW SCLC-1 und -2 nicht mehr erkannt.
   Nach Immunfärbung des Western blots mit dem MAK BW SCLC-1 können nur die Antigene mit einem Molekulargewicht von 170 ± 10 kDa, 140 ± 10 kDa und 105 ± 10 kDa nachgewiesen werden.
c) Nach Behandlung der Antigene mit Vibrio cholerae Neuraminidase (0.1 U/ml für 12 Std. bei 37°C) bzw. nach Behandlung mit NaJO₄ (1 mM; 1 h, 25°C) waren beide MAK noch in der Lage, an das Antigen zu binden. diese Befunde deuten darauf hin, daß die von MAK BW SCLC-1 und -2 definierten Epitope auf den Glykoproteinantigenen Proteinepitope darstellen.
   Diese Befunde werden dadurch erhärtet, daß die Epitope durch Proteasebehandlung zerstört werden (Pronase P; 0.1 mg/ml; 72 h, 37°C).
d) Zwei im Vergleich eingesetzte MAK gegen N-CAM (neurale Zelladhäsionsmoleküle) (Kibbelaar et al., Journal of Pathology, 159: 23 - 28, 1989) zeigten beide eine Bindung an die 170 ± 10 kDa, 140 ± 10 kDa und 105 ± 10 kDa Antigene. Der MAK 735 ist gegen α-(2-8)-verknüpfte N-Acetyl-Neuraminsäure gerichtet. Es ist anzunehmen, daß es sich bei der kleineren, präferentiell gefärbten Glykoproteinbande von 105 ± 10 kDa wahrscheinlich um die kleinere der 3 iso-Formen des N-CAM handelt, die in höheren Konzentrationen neben den größeren N-CAM iso-Formen in überständen von kleinzelligen Lungenkarzinomzellinien nachweisbar sind. Die Affinitätskonstante des MAK BW SCLC-1 wurde in einem Zellbindungsassay an 3 verschiedenen humanen Gliomzelllinien bestimmt und liegt im Bereich von 1 x 10¹⁰ M⁻¹.
e) Aus Seren von SCLC-Tumorpatienten wurde mittels Affinitätschromatographie mit dem MAK BW SCLC-1 nach SDS-PAGE unter nicht reduzierenden Bedingungen zwei Antigene mit einem Molekulargewicht von 70 - 80 und 90 - 120 kDa im Western blot nachgewiesen, bei denen es sich wahrscheinlich um iso-Formen des N-CAM handelt. Desweiteren wurde aus Seren von SCLC-Patienten mittels Affinitätschromatographie mit der MAK 735-Sepharose Antigene isoliert, die unter nicht reduzierenden Bedingungen in der SDS-PAGE ein Molekulargewicht von 220 -260 kDa und 160-200 kDa aufweisen und sich im Western blot mit dem MAK BW SCLC-1 immunchemisch anfärben lassen. Im Serum gesunder Blutspender konnten unter den gleichen Versuchsbedingungen derartige Antigene nicht bzw. nur in sehr geringen Mengen nachgewiesen werden.

Anschließend wurde mittels eines Radioimmunoassays (RIA) die Anbindung des mit J-125 markierten MAK BW SCLC-1 an je 2 in vitro kultivierte humane Melanomzell- und Neuroblastomzellinien gemessen. Es wurde festgestellt, daß der MAK bei 37°C sehr schnell an Epitop-positive Zellinien band (1 - 5 min), aber relativ schnell wieder abgegeben wurde (> 10 min bei 37°C). Bei 4°C blieb der MAK lange auf den Tumorzellen gebunden. Dieser Befund und die Präsenz der 5 zuvor beschriebenen Glykoproteine (N-CAMiso-Formen) in Überständen kleinzelliger Lungenkarzinomzellinien deuteten auf eine aktive Abgabe der Antigene durch Tumore neuroektodermalen Ursprungs hin.

Nach Biotinylierung des MAK BW SCLC-1 konnte weiterhin mittels eines Doppeldeterminantenassays gezeigt werden, daß die N-CAM Glykoproteine mindestens 2 Epitope für diesen MAK tragen.
Kompetitionsstudien mit MAK BW SCLC-2 ergaben, daß beide MAK verschiedene Epitope auf den gleichen Antigen erkennen.

### Beispiel 4

### Immunoassay zur Bestimmung des tumorassozierten Antigens in humanen Körperflüssigkeiten

Nach dem Fachmann bekannten Verfahren erfolgte die adsorptive Bindung des MAK 735 an die Polystyroloberfläche der Vertiefungen von Mikrotitrationsplatten sowie die kovalente Kopplung der MAK BW SCLC-1 und BW SCLC-2 an das Enzym Peroxidase. Zur Bestimmung der Konzentration des weiter oben beschriebenen tumorassoziierten Antigens wurden je 10 µl Probenmaterial sowie 100 µl Probenpuffer (OSND, Behringwerke) in die mit dem MAK 735 beschichteten Vertiefungen von Mikrotitrationsplatten (Nunc) pipettiert und 2 Stunden bei 37°C inkubiert.

Nach dreimaligem Waschen mit dem verdünnten Enzygnost-Waschpuffer (OSEW, BW) wurden in die einzelnen Vertiefungen je 100 µl des MAK BW SCLC-1-POD bzw. BW SCLC-2-POD-Konjugats eingefüllt. Der folgende zweistündige Inkubationsschritt bei 37°C wurde mit einem dreimaligen Waschzyklus abgeschlossen.

Für den 3. Inkubationsschritt bei Raumtemperatur wurden anschließend je 100 µl einer Puffer/ Substrat-Chromogenlösung (H₂0₂/TMB; OUVG/OUVF, BW) in die Vertiefungen pipettiert und die Enzymreaktion nach 30 min mit Enzygnost-Stopplösung (OSFA, BW) beendet. Die Extinktion der Proben wurde bei 450 nm ermittelt.

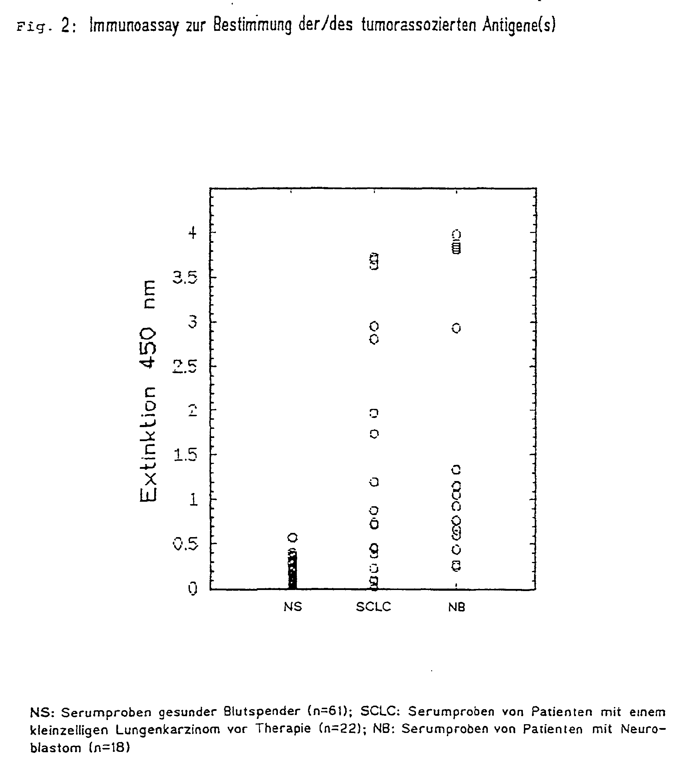

Ergebnis: Die mit diesem Immunoassay ermittelten Extinktionswerte entsprechen in ihrer Höhe der Konzentration der/des tumorassoziierten Antigene(s) in den Proben. Es zeigte sich, daß im Vergleich zu gesunden Blutspendern die Konzentration der/des tumorassoziierten Antigene(s) in den Seren von Patienten mit einem kleinzelligen Lungenkarzinom bzw. einem Neuroblastom häufig erhöht ist (Fig 2) .

Auch mit anderen Testkombinationen (z. B.: Festphasenantikörper: MAK BW SCLC-1, Konjugat: wheat germ agglutinin-POD = WGA-POD; Festphasenantikörper: MAK BW SCLC-2, Konjugat: MAK BW SCLC-1-POD) konnten höhere Antigenspiegel in Tumorseren beobachtet werden. Die Unterscheidung zwischen den Serum- oder Plasmaproben von Gesunden und denen von Patienten mit malignen Tumoren war aber nicht so ausgeprägt wie mit der weiter oben beschriebenen Testvariante.

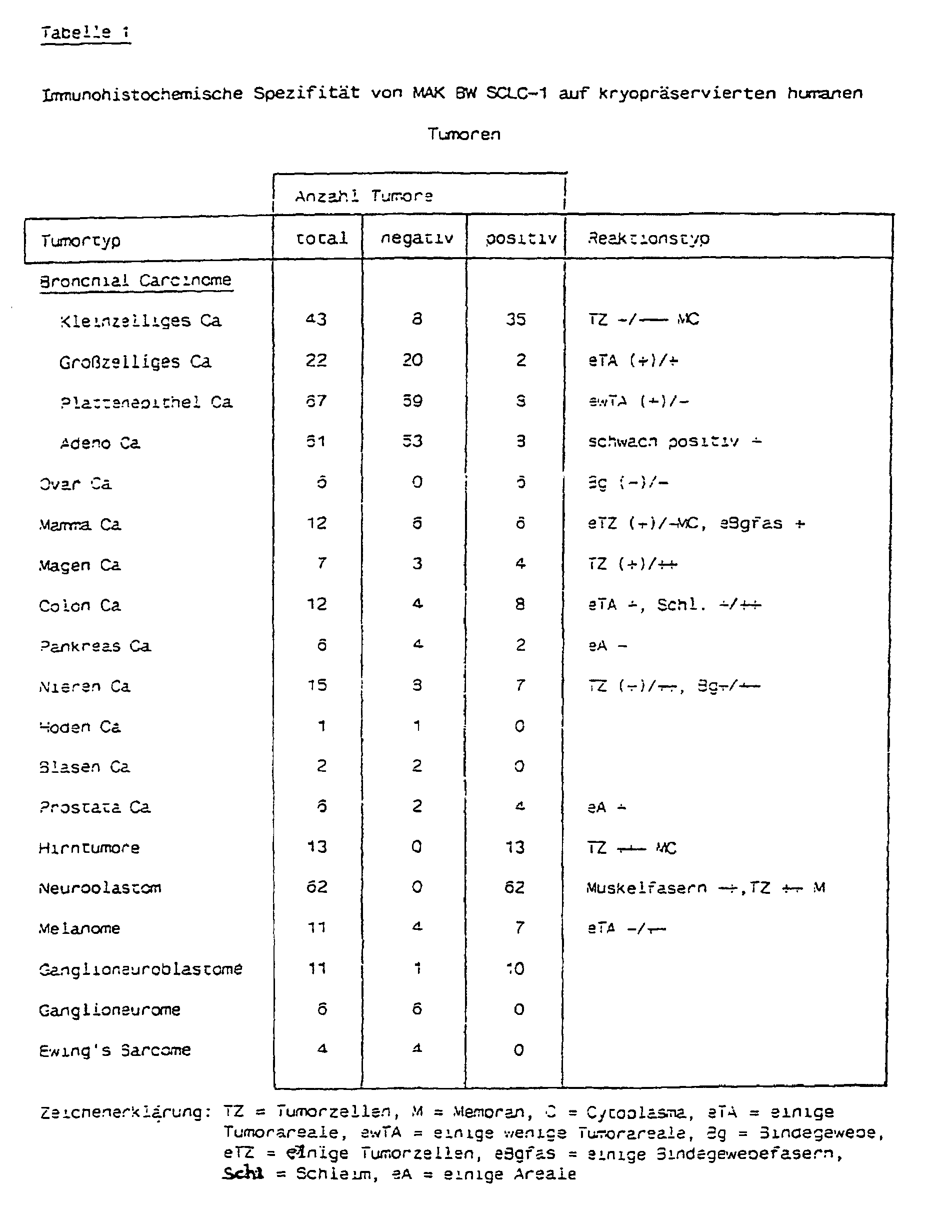

**TABELLE 2 immunhistochemische spezifität von MAK BW SCLC-1 auf Kryopräservierten humanen Normalgeweben**

| Gewebetyp | Anzahl Gewebe | | | Reaktionstyp |
|---|---|---|---|---|
| | getestet | negativ | positiv | |
| Normalgewebe: | | | | |
| Lunge | 4 | 2 | 2 | (+) eZ ++ |
| Niere | 3 | 0 | 3 | e Gef. u. Bgfas +/++ |
| Leber | 3 | 0 | 3 | Bgfas +, e Gef.Gange +/++ |
| | | | | |
| Magen | 2 | 0 | 2 | Bgfas +/++, Gef. ++ |
| Darm | 3 | 0 | 3 | Muskel (+) /+, Bgfas u. Gef.++ |
| | | | | |
| Pankreas | 3 | 0 | 3 | Inseln+, Bgfas u. Gef. ++ |
| | | | | |
| Prostata | 2 | 0 | 2 | Muskel +, homogen + |
| Mamma | 3 | 1 | 2 | Epithel ++, +/++ diff. |
| Gehirn | 9 | 0 | 9 | ++/+++ |
| Lymphknoten | 2 | 0 | 2 | eZ + MC |
| Knochenmark | 5 | 3 | 2 | ewZ + MC |
| Milz | 2 | 0 | 2 | (+), Gef. (+) /+ |
| Hoden | 1 | 0 | 1 | e Gänge (+) /++ |
| Blase | 1 | 0 | 1 | e Muskelfas +/++ |
| Nerven | 1 | 0 | 1 | Nervenfasern +++ |
| Mandeln | 1 | 0 | 1 | eBgfas+, e Muskelfasern +, ewZ +/++ |
| | | | | |
| Ovar | 1 | 0 | 1 | Epithel ++, eZ Bg ++ |
| Thymus | 1 | 0 | 1 | eZ +, Hassal-Körperchen ++ |

| Zytofluorometrische Analyse auf peripheren Blutzellen | | | | |
|---|---|---|---|---|
| Lymphozyten | 2 | * 2,8 % | 0 | Zeichenerklärungen: |
| Konozyten | 2 | * 2,5 % | 0 | eZ= einige Zellen, |
| Granulozyten | 2 | * 0,8 % | 0 | e Gef.= einige Gefäße |
| Erythrozyten | 2 | * 0,2 % | 0 | Bgfas= Bindegewebefasern |
| Thrombozyten | 2 | * 0,5 % | 0 | ewZ= einige wenige Zellen e= einige, M=Membran, C= Cytoplasma |
| | | | | * Anteil fluoreszierender Zellen, unter background. |

## Patentansprüche (Patentansprüche für folgende(n) Vertragsstaat(en): AT, BE, CH, DE, FR, GB, IT, LI, LU, NL, SE)

1. Hybridomzellinie hinterlegt unter ECACC Deposit Nr. 90 022 110

2. Monoklonaler Antikörper MAK BW SCLC-1, der von der Hybridomzellinie gemäß Anspruch 1 produziert wird.

3. In vitro Verwendung eines Monoklonalen Antikörpers gemäß Anspruch 2 als Diagnostikum.

4. Monoklonaler Antikörper gemäß Anspruch 2 zur Verwendung als Therapeutikum.

5. Spezifischer Bindungspartner, **dadurch gekennzeichnet, daß** er an dasselbe Epitop bindet wie der monoklonale Antikörper gemäß Anspruch 2.

## Patentansprüche (Patentansprüche für folgende(n) Vertragsstaat(en): ES)

1. Verwendung der Hybridomzellinie ECACC Deposit Nr. 90 022 110 zur Produktion von monoklonalen Antikörpern.

2. In vitro Verwendung des monoklonalen Antikörpers, der von der Hybridomzellinie ECACC Deposit Nr. 90 022 110 produziert wird, als Diagnostikum.

3. Verwendung des monoklonalen Antikörpers, der von der Hybridomzellinie ECACC Deposit Nr. 90 022 110 produziert wird, zur Herstellung eine Medikaments zur Verwendung als Therapeutikum

## Claims (Claims for the following Contracting State(s): AT, BE, CH, DE, FR, GB, IT, LI, LU, NL, SE)

1. A hybridoma cell line deposited under ECACC Deposit No. 90 022 110.

2. A monoclonal antibody MAb BW SCLC-1 produced by the hybridoma cell line as claimed in claim 1.

3. The in vitro use of a monoclonal antibody as claimed in claim 2 as diagnostic aid.

4. The monoclonal antibody as claimed in claim 2 for use as therapeutic agent.

5. A specific binding partner which binds to the same epitope as the monoclonal antibody as claimed in claim 2.

## Claims (Claims for the following Contracting State(s): ES)

1. The use of the hybridoma cell line deposited under ECACC Deposit No. 90 022 110 for producing monoclonal antibodies.

2. The in vitro use of the monoclonal antibody produced by the hybridoma cell line deposited under ECACC Deposit No. 90 022 110 as diagnostic aid.

3. The use of the monoclonal antibody produced by the hybridoma cell line deposited under ECACC Deposit No. 90 022 110 for producing a medicament for use as therapeutic agent.

## Revendications (Revendications pour l'(les) Etat(s) contractant(s) suivant(s): AT, BE, CH, DE, FR, GB, IT, LI, LU, NL, SE)

1. Lignée d'hybridome déposée sous le numéro de dépôt ECACC n° 90 022 110.

2. Anticorps monoclonal MAK BW SCLC-1, qui est produit par la lignée d'hybridome selon la revendication 1.

3. Utilisation in vitro d'un anticorps monoclonal selon la revendication 2, en tant qu'agent de diagnostic.

4. Anticorps monoclonal selon la revendication 2, pour utilisation en tant qu'agent thérapeutique.

5. Partenaire de liaison spécifique, **caractérisé en ce qu'**il se lie au même épitope que l'anticorps monoclonal selon la revendication 2.

## Revendications (Revendications pour l'(les) Etat(s) contractant(s) suivant(s): ES)

1. Utilisation de la lignée d'hybridome de numéro de dépôt ECACC n° 90 022 110 pour la préparation d'anticorps monoclonaux.

2. Utilisation in vitro d'un anticorps monoclonal qui est préparé à partir de la lignée d'hybridome de numéro de dépôt ECACC n° 90 022 110, en tant: qu'agent de diagnostic.

3. Utilisation d'un anticorps monoclonal qui est préparé à partir de la lignée d'hybridome de numéro de dépôt ECACC n° 90 022 110 pour la préparation d'un médicament destiné à une utilisation en tant qu'agent thérapeutique.
